# EUROPEAN PATENT SPECIFICATION

(11) **EP 3 986 390 B1**
(45) Date of publication and mention of the grant of the patent: **18.11.2026**
(21) Application number: 20825416.9
(22) Date of filing: 11.06.2020
(51) Int. Cl.: A61K 31/522, A61K 31/198, A61K 31/353, A23L 2/38, A23L 2/39, A23L 2/52, A23L 2/56, A23L 2/68, A23L 33/175, A61K 45/06, A23L 33/105

(54) **NON-VASOCONSTRICTING ENERGY-PROMOTING COMPOSITIONS CONTAINING KETONE BODIES**
NICHT-VASOKONSTRIKTIVE ENERGIEFÖRDERNDE ZUSAMMENSETZUNGEN MIT KETONKÖRPERN
COMPOSITIONS FAVORISANT L'ÉNERGIE SANS VASOCONSTRICTEUR CONTENANT DES CORPS CÉTONIQUES

(30) Priority: 21.06.2019 US 201962864989 P; 15.04.2020 US 202016849901
(43) Date of publication of application: 27.04.2022
(73) Proprietor: Axcess Global Sciences, LLC, Salt Lake City, Utah 84117 (US)
(72) Inventor: MILLET, Gary, Salt Lake City, Utah 84121 (US)
(74) Representative: Schwarz & Partner Patentanwälte GmbH
(86) International application number: PCT/US2020/037289
(87) International publication number: WO 2020/257055

(56) References cited:
- US-A1- 2007 179 197
- US-A1- 2017 020 844
- US-A1- 2018 021 274
- US-B1- 10 245 242
- US-B1- 6 835 750
- DATABASE GNPD [online] MINTEL; 11 May 2018 (2018-05-11), ANONYMOUS: "Strawberry Pineapple Flavour Pre-Exertion Performance Optimizer", XP093048093, retrieved from https://www.gnpd.com/sinatra/recordpage/5661617/ Database accession no. 5661617
- DATABASE GNPD [online] MINTEL; 29 September 2016 (2016-09-29), ANONYMOUS: "Blue Lemon Ice Advanced Formula", XP093048090, retrieved from https://www.gnpd.com/sinatra/recordpage/4315637/ Database accession no. 4315637
- MANGELS DANIEL R. ET AL: "Catechins as Potential Mediators of Cardiovascular Health", TRANSLATIONAL SCIENCES, vol. 37, no. 5, 1 May 2017 (2017-05-01), pages 757 - 763, XP093048532, ISSN: 1079-5642, DOI: 10.1161/ATVBAHA.117.309048
- KASTER MANUELLA P. ET AL: "Caffeine acts through neuronal adenosine A 2A receptors to prevent mood and memory dysfunction triggered by chronic stress", PROCEEDINGS OF THE NATIONAL ACADEMY OF SCIENCES, vol. 112, no. 25, 8 June 2015 (2015-06-08), pages 7833 - 7838, XP093048389, ISSN: 0027-8424, DOI: 10.1073/pnas.1423088112
- AMAZON: "Perfect Keto Perform Pre Workout Powder - Burn Fat for Fuel Energy Supplement Drink Mix for Men and Women - Keto Friendly with Ketone Salts, BCAA, Nitric Oxide & MCT", 25 September 2017 (2017-09-25), pages 1, XP055776200, Retrieved from the Internet <URL:https://www.amazon.com/Perfect-Keto-Perform-Preworkout-Supplement/dp/B0751379Q9/ref=sr_1_9?dchild=1&keywords=ketone+pre+workout&qid=1597938465&sr=8-9>
- S G HOLTZMAN, S MANTE AND K P MINNEMAN: "Role of adenosine receptors in caffeine tolerance", JOURNAL OF PHARMACOLOGY AND EXPERIMENTAL THERAPEUTICS, vol. 256, no. 1, 1 January 1991 (1991-01-01), US , pages 62 - 68, XP009532712, ISSN: 0022-3565

## Description

### BACKGROUND

Energy drinks, such as ketoperform or bpi sports, are beverages used by consumers to promote wakefulness, maintain alertness, and enhance mood and cognition. Typical gas station energy drinks include common ingredients such as caffeine and sugar. Some may also include vitamins, electrolytes, or amino acids.

Despite widespread use, there are several health concerns surrounding consumption of gas station energy drinks, particularly surrounding their effects on the cardiovascular system. Such energy drinks have been shown to increase heart rate, increase blood pressure, and even thicken the blood. Energy drinks may also affect the brain in undesirable ways, such as by raising stress levels and inducing anxiety, insomnia, gastrointestinal irritation, muscle twitching, and periods of prolonged restlessness.

Energy drinks are also associated with high levels of fatigue, irritability, and sub-optimal cognition following the initial boost after consumption. This energy "crash" can be long lasting and unpleasant. As the stimulating effects wear off, levels of adrenaline, dopamine, and acetylcholine begin to drop, and blood sugar levels may swing erratically, often leaving the user more fatigued, irritable, and unfocused than before consuming the energy drink. In the case of caffeine in particular, the user's adenosine levels may continue to rise after dosing. When caffeine is no longer available to block adenosine receptors, the rapid flood of built up adenosine to the receptors can contribute to the perceived crash.

To date, popular gas station energy drinks have generally failed to improve the overall quality of life and well-being of users. Rather, such energy drinks provide a short-lived boost in desired effects but with a subsequent crash that often tends to be so uncomfortable as to negate the benefits and place consumption of energy drinks as a net negative experience. When used to stay awake while driving, gas station energy drinks can cause high levels of stress and possibly contribute to "road rage". For long haul drives, the energy "crash" is potential harmful or even fatal if it leads to a highway crash.

Accordingly, there is an ongoing need for compositions that are capable of promoting a natural and sustained feeling of wakefulness, maintaining a natural feeling of alertness without jitteriness, increasing concentration, enhancing mood, and/or enhancing cognition without the typical energy crash and without damaging the long-term physiological or mental health of the user.

### SUMMARY

The present invention is defined by the appended claims. Any subject-matter falling outside the scope of the claims is provided for information only.

Disclosed herein are energy-promoting compositions formulated to provide rapid and effective onset of physical and mental energy, while minimizing or avoiding typical crash effects that plague conventional energy-promoting products, particularly gas station energy drinks advertised on billboards, radio, and television. In one embodiment, the energy-promoting composition is formulated as a liquid energy drink. Other embodiments may include other dosage forms.

In one embodiment, a composition for promoting or supporting energy of a subject, comprising: (i) a stimulant component comprising one or more epinephrine and/or norepinephrine promoters selected from the group consisting of yohimbine, rauwolscine, ephedra-based compound, ephedrine, pseudoephedrine, synephrine, octopamine, 1,3-dimethylamylamine, higenamine, clenbuterol, hordenine, atomoxetine, and combinations thereof, or comprising one or more xanthine alkaloids selected from the group consisting of caffeine, theacrine, theophylline, methylliberine, and combinations thereof; (ii) a vasodilator component comprising one or more of quercetin, L-arginine and/or salt thereof, agmatine and/or salt thereof, gallic acid, coenzyme Q10 (CoQ10), trimethylglycine, niacin, theanine, theobromine, garlic, grape seed extract, green tea extract, catechins and/or epicatechins, proanthocyanidins, galloylated proanthocyanidins, L-norvaline, glutathione, and taurine; and (iii) an energy source comprising an exogenous ketone body component, wherein the exogenous ketone body component comprises a mixed salt-acid including beta-hydroxybutyric acid and at least one beta-hydroxybutyrate salt. The energy-promoting composition may further optionally comprise (iv) a nootropic component, such as a dopamine and/or acetylcholine promoter.

The stimulant component may comprise caffeine. The stimulant component may additionally or alternatively include other stimulant compounds described herein, epinephrine and/or norepinephrine promoters or combinations thereof. Caffeine is a preferred stimulant component because it is widely utilized, well understood, well tolerated, easy to dose, and familiar to almost all users.

The vasodilator component may comprise one or more of quercetin, L-citrulline and/or salt thereof, L-arginine and/or salt thereof, agmatine and/or salt thereof, nitrates, gallic acid, coenzyme Q10 (CoQ10), trimethylglycine, niacin, theanine, theobromine, garlic, magnesium, grape seed extract, green tea extract, catechins and/or epicatechins (e.g., Vaso 6), cacao, proanthocyanidins, galloylated proanthocyanidins, L-norvaline, beta-alanine, glutathione, and taurine. In some embodiments, the vasodilator component is a (-)-epicatechin such as epicatechin-(4-8)-epicatechin-(4-8)-epicatechin or epicatechin-(4-8)-epicatechin-(4-8)-epicatechin-gallate. L-citrulline and/or salts thereof, nitrates, cacao and beta-alanine do not fall within the scope of the claimed subject-matter.

The energy source comprises an exogenous ketone body component, namely beta-hydroxybutyrate. The ketone body component may be provided as a salt, ester, free acid (i.e., beta-hydroxybutyric acid), or combination thereof. It may also include a ketone body precursor, such as 1,3-butanediol or ester thereof.

The individual components of the energy-promoting composition beneficially and synergistically interact together to enhance the overall energy-promoting effect of the composition and provide a more naturally feeling energy boosting effect compared to popular gas station energy drinks. The combination of a stimulant component, vasodilator component, and exogenous ketone body component in a single formulation provides benefits not obtainable from a composition that includes only one or two of the components.

The vasodilator component beneficially counteracts the vasoconstriction of the stimulant component and also functions to improve the pharmacokinetic distribution of the other components. In other words, the vasodilator component functions to increase blood flow to peripheral tissues of the body so that the ketone bodies and the stimulant compound(s) can more effectively move from the digestive system to their intended targets (e.g., brain and muscle tissues).

The stimulant component beneficially interacts with the ketone body component by enhancing pharmacokinetic absorption and uptake of the ketone bodies into the tissues so it can be readily utilized as an energy source. In other words, the stimulant component has been found to increase the efficiency in which the ketone body component is actually taken into cells and metabolized as an energy source.

The ketone body component attenuates the crash effect of the stimulant component by beneficially smoothing out and/or prolonging the experienced energy levels of the user. For example, the ketone body component provides a stable fuel source that avoids or minimizes the blood sugar swings and/or insulin spikes and/or mood swings commonly associated with conventional energy drinks.

It is to be understood that both the foregoing general description and the following detailed description are exemplary and explanatory only and are not restrictive of the embodiments claimed.

### BRIEF DESCRIPTION OF THE DRAWINGS

Various objects, features, characteristics and advantages of the invention will become apparent and more readily appreciated from the following description of the embodiments, taken in conjunction with the accompanying drawings and the appended claims, all of which form a part of this specification wherein like reference numerals designate corresponding parts in the various figures and wherein the various elements depicted are not necessarily drawn to scale; and wherein:

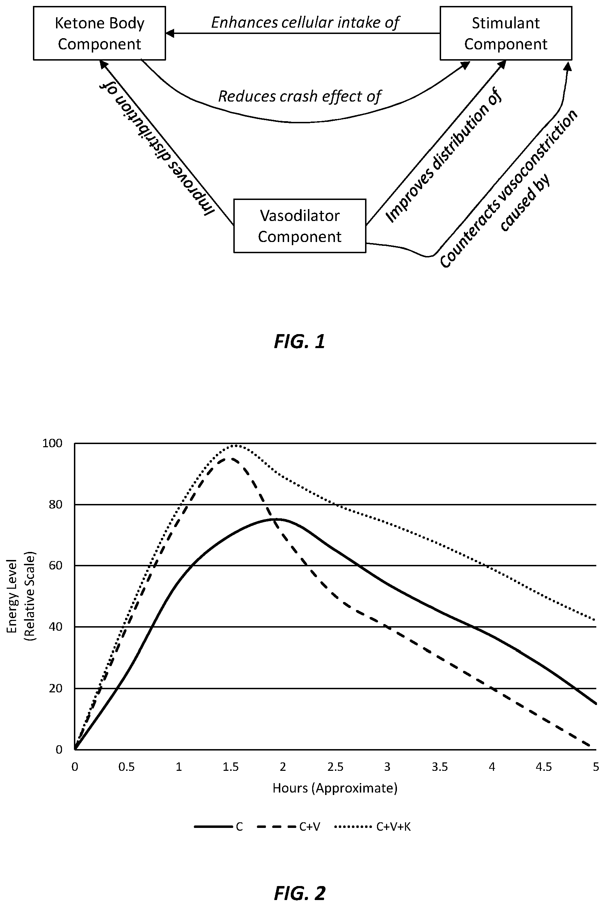

Figure 1 is a flow diagram that schematically illustrates the multiple interactions between the individual stimulant, vasodilator, and ketone body components of an energy-promoting composition; and
Figure 2 is a chart that graphically illustrates the expected differences in energy levels experienced by a typical user when consuming a composition including caffeine ("C"), an otherwise similar composition including caffeine and a vasodilator component ("C+V"), and an otherwise similar composition including caffeine, a vasodilator component, and a ketone body component ("C+V+K") according to the invention.

### DETAILED DESCRIPTION

### I. Definitions

As used herein, the term "crash" or "crash effect" refers to the state a user may experience following consumption of a stimulant such as caffeine. A crash is characterized by unpleasant effects such as extreme tiredness, difficulty concentrating, irritability, diminished judgment, and/or poor mood. As the stimulant wears off, neurotransmitter and/or hormone (e.g., dopamine, acetylcholine, adrenaline) levels may drop relatively sharply, and blood sugar level swings may become larger than normal. Insulin spikes may also exacerbate the crash. In the case of caffeine in particular, adenosine levels may continue to rise after dosing. When caffeine is no longer available to block adenosine receptors, the rapid flood of built up adenosine to the receptors can contribute to the crash.

As used herein, the term "energy level," "experienced energy level," or "subjective energy level" refers to the level of wakefulness and/or focus experienced by the user. They can be both mental and physical in nature. While these may be relatively subjective properties, they are readily understood by users and are amenable to measurement through appropriate questioning and/or surveying. For example, as with various clinical mood or pain rating measurements, a user may describe energy level on a scale (e.g., 1 to 10 or 1 to 100). Other objective measures of wakefulness are also known in the art, such as those described in Michael et al. (2008) "Effects of caffeine on alertness as measured by infrared reflectance oculography" Psychopharmacology, 200: 255-260.

The term "administration" or "administering" is used herein to describe the process in which an energy-promoting composition is taken by or delivered to a subject. The composition may be administered in various ways including orally, rectally, or intragastrically, among others, though typical embodiments will be formulated for oral administration.

The term "dosage form" refers to the structural form the composition is provided in and/or the vehicle by which the autobiotic composition is delivered to the user. Example dosage forms include, but are not limited to, tablets, capsules, powders, gels, food products, food additives, beverages/drinks (e.g., in cans, bottles, cartons, pouches, squeeze containers, and the like), beverage additives, candies (e.g., suckers, gummies, pastilles), food supplements, sprays, injectables, and suppositories. Preferred embodiments are formulated as liquids for oral consumption.

The term "unit dose" refers to a dosage form that is configured to deliver a specified quantity or dose of composition or component thereof. Dosage forms may be configured to provide a full unit dose or fraction thereof (e.g., 1/2, 1/3, or 1/4 of a unit dose).

Another dosage form that can be used to provide a unit dose of composition or component thereof is a "unit dose measuring device", such as a cup, scoop, syringe, dropper, spoon, spatula, or colonic irrigation device, which is configured to hold therein a measured quantity of composition equaling a full unit dose or fraction thereof (e.g., 1/2, 1/3, or 1/4 of a unit dose). An example use of such measuring device(s) is in kit together with a bulk container.

For example, a "bulk container", such as a carton, box, can, jar, bag, pouch, bottle, jug, or keg, containing several unit doses of composition (e.g., 5-250 or 10-150 unit doses) can be provided to a user together with a unit dose measuring device that is configured to provide a unit dose, or fraction thereof, of composition or component thereof.

A "kit" for use in providing a composition as disclosed herein in bulk form, while providing unit doses of the composition, may comprise a bulk container holding therein a quantity of composition and a unit dose measuring device configured to provide a unit dose, or fraction thereof, of composition or component thereof. One or more unit dose measuring devices may be positioned inside the bulk container at the time of sale, attached to the outside of the bulk container, prepackaged with the bulk container within a larger package, or provided by the seller or manufacture for use with one or multiple bulk containers.

The kit may include instructions regarding the size of the unit dose, or fraction thereof, and the manner and frequency of administration. The instructions may be provided on the bulk container, prepackaged with the bulk container, placed on packaging material sold with the bulk container, or otherwise provided by the seller or manufacturer (e.g., on websites, mailers, flyers, product literature, etc.) The instructions for use may include a reference on how to use the unit dose measuring device to properly deliver a unit dose or fraction thereof. The instructions may additionally or alternatively include a reference to common unit dose measuring devices, such as spoons, spatulas, scoops, droppers, cups, syringes, colonic irrigation device, and the like, not provided with the bulk container (e.g., in case the provided unit dose measuring device is lost or misplaced). In such case, a kit may be constructed by the end user when following instructions provided on or with the bulk container, or otherwise provided by the seller regarding the product and how to properly deliver a unit dose of composition, or fraction thereof.

The term "exogenous ketone body" refers to beta-hydroxybutyrate. This compound may be utilized by a subject's body as an energy source during instances of low glucose levels or when these compounds are supplemented in a usable form. Where beta-hydroxybutyrate is included, it may be provided as purified or enriched with the R enantiomer, as purified or enriched with the S enantiomer, or as a racemic mixture. The exogenous ketone bodies may be provided in a free acid form (i.e., beta-hydroxybutyrate), salt form, ester form, or combination thereof. Ketone body precursors, such as 1,3-butanediol or ester thereof can be included.

### II. Energy-Promoting Compositions

In one embodiment, a composition for promoting or supporting energy of a subject, comprising: (i) a stimulant component comprising one or more epinephrine and/or norepinephrine promoters selected from the group consisting of yohimbine, rauwolscine, ephedra-based compound, ephedrine, pseudoephedrine, synephrine, octopamine, 1,3-dimethylamylamine, higenamine, clenbuterol, hordenine, atomoxetine, and combinations thereof, or comprising one or more xanthine alkaloids selected from the group consisting of caffeine, theacrine, theophylline, methylliberine, and combinations thereof; (ii) a vasodilator component comprising one or more of quercetin, L-arginine and/or salt thereof, agmatine and/or salt thereof, gallic acid, coenzyme Q10 (CoQ10), trimethylglycine, niacin, theanine, theobromine, garlic, grape seed extract, green tea extract, catechins and/or epicatechins, proanthocyanidins, galloylated proanthocyanidins, L-norvaline, glutathione, and taurine; and (iii) an energy source comprising an exogenous ketone body component, wherein the exogenous ketone body component comprises a mixed salt-acid including beta-hydroxybutyric acid and at least one beta-hydroxybutyrate salt. The energy-promoting composition may further optionally comprise (iv) a nootropic component such as a dopamine and/or acetylcholine promoter.

### A. Stimulant Component

In some embodiments, the stimulant component comprises an effective amount of caffeine. The caffeine may be included in an amount ranging from about 30 mg to about 250 mg, or about 40 mg to about 225 mg, or about 50 mg to about 200 mg, or about 60 mg to about 180 mg, or about 80 mg to about 160 mg. Caffeine is a preferred stimulant component because it is widely utilized, well understood, well tolerated, easy to dose, and familiar to almost all users.

Unfortunately, caffeine also causes vasoconstriction and other associated effects such as increased blood pressure, which are undesirable for the typical, casual user. As with other stimulants, caffeine also tends to have a crash effect where users feel tired and sluggish as the dose is metabolized and wears off.

The stimulant component may additionally or alternatively include other stimulant compounds, such as one or more epinephrine and/or norepinephrine promoters, or combinations thereof. Where such additional or alternative stimulant compounds are utilized, they are preferably provided in an amount such that the combined stimulant composition provides a stimulant effect substantially similar to that provided by caffeine in amounts within the ranges described above.

In some embodiments, the stimulant component includes other xanthine alkaloids in addition to or alternative to caffeine, such as theacrine, theobromine, theophylline, methylliberine, or combinations thereof. While most of these compounds are generally effective stimulants, most are also associated with undesirable vasoconstriction and potential increases in blood pressure, as with caffeine. As an exception, theobromine functions as a very mild vasodilator. Theacrine and methylliberine are believed to be somewhat less vasoconstricting than caffeine, though neither are vasodilators and at best still likely have a mild vasoconstricting effect. Theacrine is often confused with the amino acid theanine, which in contrast does promote vasodilation. An example of methylliberine is Dynamine^{™} manufactured by Compound Solutions, Inc.

Epinephrine and/or norepinephrine promoters, if utilized in the stimulant component, may include one or more of yohimbine, rauwolscine, *Ephedra-*based compounds such as ephedrine and pseudoephedrine, synephrine, octopamine, 1,3-dimethylamylamine, higenamine, clenbuterol, and adrenaline/noradrenaline reuptake inhibitors , namely hordenine and atomoxetine.

### B. Vasodilating Component

In some embodiments, the vasodilator component comprises one or more of quercetin, L-citrulline and salts thereof such as citrulline malate, L-arginine and salts thereof, agmatine and salts thereof, nitrates (extracts from beetroot, celery, cress, chervil, lettuce, spinach, arugula), gallic acid, coenzyme Q10 (CoQ10), trimethylglycine, niacin, theanine, theobromine, garlic, magnesium, grape seed extract, green tea extract, catechins and/or epicatechins (e.g., from green tea or and/or grape seed), proanthocyanidins (e.g., galloylated proanthocyanids), arginase inhibitors such as L-norvaline, nitric oxide synthase promoters such as beta-alanine and/or glutathione, and GABA receptor agonists such as taurine. Many of the foregoing vasodilating compounds function at least partially as nitric oxide promoters, though others may involve other primary vasodilating mechanisms. L-citrulline and/or salts thereof, nitrates, cacao and beta-alanine do not fall within the scope of the claimed subject-matter.

In general, example monomer catechins and epicatechins have the following formulas and are stereoisomers of each other:

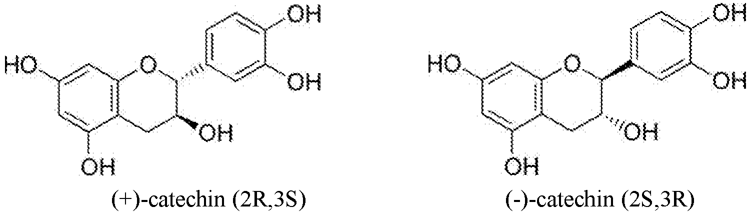

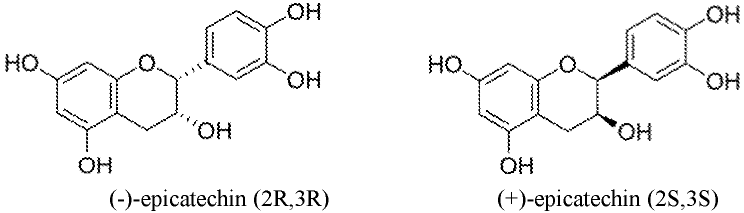

Catechins and epicatechins have vasodilating properties and can be used in any combination or derivative, including dimers, trimers, and oligomers thereof. In some embodiments, the vasodilator component includes (-)-epicatechin derivatives, such as epicatechin-(4-8)-epicatechin-(4-8)-epicatechin or epicatechin-(4-8)-epicatechin-(4-8)-epicatechin-gallate, the latter of which is depicted below. Such compounds are described in detail in U.S. Patent No. 6,706,756. An example of epicatechin-containing supplement is Vaso 6^{™} manufactured by Compound Solutions, Inc.

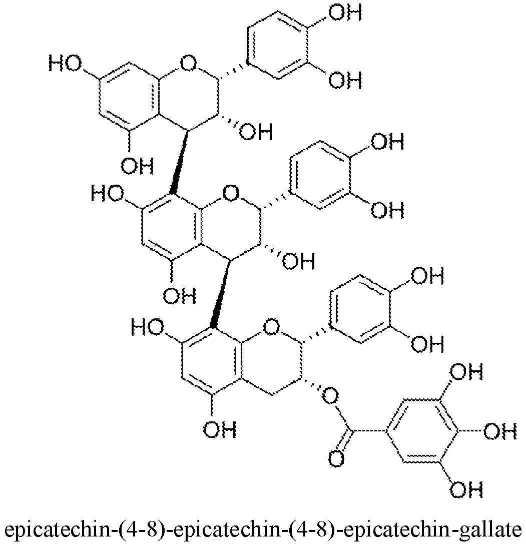

The amount of the vasodilating component included may be varied according to the particular combination of compounds making up the vasodilator component. In some embodiments, the vasodilating component is provided at a functional dose formulated to provide a vasodilating effect substantially similar to a dose of about 1 g to about 8 g citrulline, or about 2 g to about 6.5 g citrulline, or about 3 g to about 5 g citrulline.

Several techniques are known in the art for measuring vasodilation, including those described by Lind et al. "Evaluation of four different methods to measure endothelium-dependent vasodilation in the human peripheral circulation" Clin Sci (Lond), 2002 May; 102(5):561-7; Heiss et al. "In vivo measurement of flow-mediated vasodilation in living rats using high-resolution ultrasound" Am J Physiol Heart Circ Physiol., 2008 Feb; 294(2):H1086-93; and Yamashita et al. "The effects of vasodilation on cardiac output measured by PiCCO" J Cardiothorac Vasc Anesth. 2008 Oct; 22(5): 688-92, for example.

### C. Ketone Bodies

The energy source comprises an exogenous ketone body component such as beta-hydroxybutyrate and/or acetoacetate. The ketone body component may be provided as a salt, ester, free acid (i.e., beta-hydroxybutyric acid), or combination thereof. A ketone body precursor such as 1,3-butanediol or ester thereof may optionally be included. Exogenous ketone bodies are particularly beneficial for supporting stable energy levels without excessive "jitters" and without a rapid crash following consumption. The brain can utilize ketone bodies as an energy source, and in many instances, ketone bodies are the preferred source of energy for brain cells. An increase in the proportion of the brain's energy demand met by ketone bodies, as opposed to glucose, beneficially provides a stable and relatively long-lasting energy source to fuel the brain. Ketone bodies can provide an elevated fuel source without causing an insulin spike, as is common with rapid consumption of sugars.

"Beta-hydroxybutyrate," also known as β-hydroxybutyrate, 3-hydroxybutyrate, βHB, or BHB, is the deprotonated form of beta-hydroxybutyric acid, a hydroxycarboxylic acid having the general formula CH₃CH₂OHCH₂COOH. The deprotonated form present at typical biological pH levels is CH₃CH₂OHCH₂COO⁻. The general chemical structure shown below represents beta-hydroxybutyrate compounds that may be utilized in the disclosed compositions: 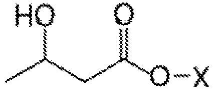 where,
X can be hydrogen, metal ion, amino cation such as from an amino acid, alkyl, alkenyl, aryl, or acyl.

When X is a hydrogen, the compound is beta-hydroxybutyric acid. When X is a metal ion or an amino cation, the compounds is a beta-hydroxybutyrate salt. When X is alkyl, alkenyl, aryl, or acyl, the compounds is a beta-hydroxybutyrate ester. The foregoing compounds can be in any desired physical form, such as crystalline, powder, solid, liquid, solution, suspension, or gel.

The beta-hydroxybutyrate and acetoacetate compounds described above may be collectively referred to herein as "ketone bodies," "exogenous ketone bodies," a "ketone body component," or "exogenous ketones."

The term "beta-hydroxybutyrate mixed salt-acid composition" means a composition that includes one or more salts of beta-hydroxybutyrate and free beta-hydroxybutyric acid.

The terms "beta-hydroxybutyrate salt" and "acetoacetate salt" do not mean or imply any particular physical state, such as a crystalline, powder, other solid form, dissolved in water to form a liquid solution, dispersed in a liquid to form a suspension, or a gel. A salt can be formed in solution by neutralizing beta-hydroxybutyric acid and/or acetoacetic acid with a strong or weak base, such as an alkali or alkaline earth metal hydroxide, carbonate, or bicarbonate, basic amino acid, and the like.

The term "free beta-hydroxybutyric acid" means the sum of non-deprotonated and deprotonated beta-hydroxybutyric acid molecules. A deprotonated beta-hydroxybutyric acid molecule generally means a molecule that has released a proton to form a hydronium ion (H₃O+) and a beta-hydroxybutyrate anion (e.g., dissolved in water).

Free beta-hydroxybutyric acid molecules are typically not deprotonated to any significant degree when contained in a beta-hydroxybutyrate mixed salt-acid composition in dry powder or other solid form. In such cases, the fractional amount of free beta-hydroxybutyric acid in a beta-hydroxybutyrate mixed salt-acid composition on a weight basis is the weight of free beta-hydroxybutyric acid divided by the combined weight of free beta-hydroxybutyric acid and beta-hydroxybutyrate salt(s). On a molar basis, the fractional amount of free beta-hydroxybutyric acid in an beta-hydroxybutyrate mixed salt-acid composition are the molar equivalents of free beta-hydroxybutyric acid divided by the sum of molar equivalents of free beta-hydroxybutyric acid and beta-hydroxybutyrate anions provided by the beta-hydroxybutyrate salt(s). Similar principles apply to acetoacetic acid.

When dissolved in water, a portion of the beta-hydroxybutyric acid will typically dissociate into beta-hydroxybutyrate anions and hydronium ions (H₃O+). As a result, beta-hydroxybutyric acid molecules can exchange protons and cations with dissolved beta-hydroxybutyrate salts. For purposes of defining the relative amounts of beta-hydroxybutyric acid and beta-hydroxybutyrate salt(s) in a beta-hydroxybutyrate mixed salt-acid composition, dissociation of beta-hydroxybutyric acid molecules and the exchange of protons and cations is not understood as changing the molar ratio of free beta-hydroxybutyric acid relative to beta-hydroxybutyrate anions from the beta-hydroxybutyrate salt(s). The total quantity of free beta-hydroxybutyric acid molecules in solution is the sum of dissolved beta-hydroxybutyric acid molecules that are not deprotonated and beta-hydroxybutyrate anions formed by deprotonation of beta-hydroxybutyric acid molecules. Similar principles apply to acetoacetic acid.

Stated another way, the total molar equivalents of beta-hydroxybutyric acid in solution, whether or not deprotonated, is understood to be the difference between (i) the sum of molar equivalents of non-deprotonated beta-hydroxybutyric acid molecules and total molar equivalents of beta-hydroxybutyrate anions in solution (from all sources) and (ii) the total molar equivalents of cationic charge provided by cations from the beta-hydroxybutyrate salt compounds (which equals the total molar equivalents of beta-hydroxybutyrate anions provided by the beta-hydroxybutyrate salt(s)). Alkali metal cations such as sodium and potassium provide 1 mole of cationic charge per mole of metal cations. Alkaline earth metal cations such as magnesium and calcium, on the other hand, provide 2 moles of cationic charge per mole of metal cations. 1 mole of deprotonated beta-hydroxybutyric acid molecules provide 1 mole of anionic charge and one mole of cationic charge. Similar principles apply to acetoacetic acid.

In view of the foregoing, the molar fraction of beta-hydroxybutyric acid in solution in relation to total moles of beta-hydroxybutyrate molecules from the beta-hydroxybutyrate mixed salt-acid composition in solution is [(i) - (ii)] ÷ (i), and the molar fraction of beta-hydroxybutyrate molecules from the beta-hydroxybutyrate salt(s)) in solution is (ii) ÷ (i). Multiplying the molar fraction of each by 100 gives the percentage of each in solution. Similar principles apply to acetoacetate mixed salt-acid compositions.

By way of example, if 100 molar equivalents of beta-hydroxybutyrate mixed salt-acid composition in a dry powdered state contained 5% of free non-deprotonated beta-hydroxybutyric acid and 95% beta-hydroxybutyrate salt(s) on a molar basis, there would be essentially 5 molar equivalents of beta-hydroxybutyric acid molecules and 95 molar equivalents of beta-hydroxybutyrate anions. When there is sufficient water to dissolve the beta-hydroxybutyrate salt(s), and if a portion of the beta-hydroxybutyric acid molecules were deprotonated, the molar equivalents of non-deprotonated beta-hydroxybutyric acid would be less than 5 and the molar equivalents of beta-hydroxybutyrate anions would be greater than 95. The extent of deprotonation of beta-hydroxybutyric acid in solution is related to solution pH. Similar principles apply to acetoacetate mixed salt-acid compositions.

Beta-hydroxybutyrate mixed salt-acid compositions, when used, contain one or more beta-hydroxybutyrate salts and beta-hydroxybutyric acid. Providing a quantity of beta-hydroxybutyric acid is beneficial because of its much quicker absorption response time compared to salt forms of beta-hydroxybutyrate. Moreover, even though free beta-hydroxybutyric acid is ordinarily a liquid that forms an acidic solution of low pH when mixed in water and has an unpalatable taste, when combined with salt forms of beta-hydroxybutyrate and where the amount of beta-hydroxybutyric acid is relatively small relative to the salt form(s), the composition can still form a solid, powder or other form typical of salt forms. In such case, the combined salt and acid forms of BHB have acceptable pH and taste. beta-hydroxybutyrate mixed salt-acid compositions have substantial advantages over pure beta-hydroxybutyrate salts, including increased absorption rate, increased bioavailability, lower electrolyte load, ease of manufacture, significantly improved taste, and reduced need for citric acid or other edible acids to obtain a composition with neutral or acidic pH (e.g., compositions made acidic to preserve and prevent the compositions from supporting microbial growth). Similar principles apply to acetoacetate mixed salt-acid compositions.

In some embodiments, the beta-hydroxybutyrate mixed salt-acid composition contains less than 100% of one or more beta-hydroxybutyrate salts and greater than 0% of free beta-hydroxybutyric acid. Beta-hydroxybutyrate mixed salt-acid compositions may contain, on a molar basis, up to 99.9%, 99.8%, 99.7%, 99.6%, 99.5%, 99.4%, 99.3%, 99.2%, 99.1%, 99%, 98.8%, 98.65%, 98.5%, 98.35%, 98.2%, 98%, 97.75%, 97.5%, 97.25%, or 97%, and at least 75%, 80%, 85%, 90%, 92%, 94%, 95%, 96%, or 97%, of one or more beta-hydroxybutyrate salts and at least 0.1%, 0.2%, 0.3%, 0.4%, 0.5%, 0.6%, 0.7%, 0.8%, 0.9%, 1%, 1.2%, 1.35%, 1.5%, 1.65%, 1.8%, 2%, 2.25%, 2.5%, 2.75%, or 3%, and less than 25%, 20%, 15%, 10%, 8%, 6%, 5%, 4%, or 3%, of free beta-hydroxybutyric acid. The foregoing percentages are expressed on a molar basis (e.g., moles of beta-hydroxybutyric acid relative to total moles of beta-hydroxybutyrate compounds in both salt and acid forms). Similar principles apply to acetoacetate mixed salt-acid compositions and percentages.

The ketone body component may be included in an amount ranging from about 0.5 g to about 20 g, or about 1 g to about 15 g, or about 2 g to about 10 g, or about 3 g to about 8 g. The ketone body component is preferably comprised of a beta-hydroxybutyrate mixed acid-salt composition, such as a mixture of beta-hydroxybutyrate salt(s) and beta-hydroxybutyric acid, preferably including a mixture of beta-hydroxybutyrate salt(s), though ketone body forms may be additionally included, as mentioned above, such as acetoacetate compounds.

When a salt mixture is utilized in the ketone body component, it is preferred that the salt mixture limit the amount of sodium ions to avoid exacerbating or causing high blood pressure or other electrolyte imbalance. As explained above, the stimulant component is already associated with causing increased blood pressure. Although the vasodilator component beneficially counteracts at least some of these effects, excessive sodium ions in the blood may undo the benefits of the vasodilator component and/or promote other negative health effects related to electrolyte imbalances and particularly sodium/potassium imbalances. In some embodiments, the total amount of sodium provided by a daily dose of the energy-promoting composition does not exceed four times the recommended daily allowance (RDA) for sodium, which in the United States is currently 2,400 mg for an adult. Preferably the amount of sodium is no more than three times the RDA, more preferably no more than two times the RDA, and most preferably no more than the RDA for sodium.

In preferred embodiments, the energy-promoting composition is formulated such that the molar ratio of sodium ions to potassium ions is no greater than about 2.5, more preferably no greater than about 2.0, or about 1.5, or even more preferably no greater than about 1.0.

An example of a supplement for providing ketone bodies is goBHB^{™} manufactured by Compound Solutions, Inc.

### D. Other Components and Aspects of Composition

In some embodiments, the energy-promoting composition omits glucose or other sugars, such as fructose, sucrose, or other simple sugars that the body readily converts to glucose. Excess glucose can rapidly take the user out of ketosis, where the ketone bodies are primarily used as the cellular fuel, and causes a return to glycolysis, where glucose is the primary cellular fuel. In glycolysis, the user may be unable to reap the full benefits of the ketone body energy source. Rather, the crash effect may be exacerbated by the postprandial hypoglycemia (i.e., "sugar crash").

The energy-promoting composition may optionally include a nootropic component formulated to promote cognition, mood, and/or focus, and can thereby also aid in attenuating the crash effect of the stimulant component, in particular the drop in levels of dopamine and/or acetylcholine. In some embodiments, the nootropic component may include one or more of a dopamine promoter (including dopamine precursors, dopamine receptor agonists and/or dopamine breakdown or reuptake inhibitors), an acetylcholine promoter (including acetylcholine precursors, acetylcholine receptor agonists and/or acetylcholine reuptake inhibitors), and/or other nootropic compound(s).

A suitable dopamine promoter may include methylliberine, theacrine, *Mucuna pruriens,* tyrosine, L-DOPA (i.e., L-3,4-dihydroxyphenylalanine), phenylalanine, phenylethylamine, tolcapone, or a combination thereof. A suitable acetylcholine promoter may include huperzine-A (toothed clubmoss extract (aerial parts) from huperzia serrata), acetylcholinesterase inhibitor), dimethylaminoethanol, choline, alpha-glycerophosphocholine, uridine, citicoline, racetams such as piracetam, phenylpiracetam, oxiracetam, and aniracetam, or a combination thereof.

The nootropic component may additionally or alternatively include one or more other nootropic compounds such as a serotonin promoter (e.g., tryptophan, 5-hydroxytryptophan (5-HTP)), beta-alanine, D-serine, phosphatidylserine, *Panax ginseng, Ginkgo biloba, Rhodiola rosea, Polygala tenuifolia, Muira puama, Eschscholzia californica, Convolvulus pluricaulis, Centella asiatica, Evolvulus alsinoides, Bacopa monnieri, Epimedium* herbs, *Ashwagandha* herbs, a cyclic adenosine monophosphate (cAMP) modulator such as forskolin or vinpocetine, or a combination thereof.

The energy-promoting composition may optionally include a medium chain fatty acid, salt, or ester thereof in as a supplementary source of endogenous ketone bodies in addition to beta-hydroxybutyrate supplementation. Medium chain fatty acids, salts, or mono-, di-, or triglycerides thereof, can be converted by the body into endogenous ketone bodies. Medium chain fatty acids have from 6 to 12 carbons, preferably from 8 to 10 carbons (e.g., caproic acid, caprylic acid, capric acid, and lauric acid).

Though possibly adversely affecting taste, the energy-promoting composition may optionally include a short chain fatty acid, salt or ester thereof, or a mono-, di- or triglyceride of the at least one short chain fatty acid. Short chain fatty acids have less than 6 carbons (e.g., acetic acid, propionic acid, butyric acid, isobutyric acid, valeric acid, and isovaleric acid). An example short chain triglyceride is tributyrin. Such molecules can provide protection to the gut and improve microbiome health.

The energy-promoting compositions may include flavorings that enhance the taste. Examples include sweeteners (e.g., stevia extract, such as Rebaudioside A), fruit flavors, mint flavors, other natural flavors, and the like.

Other optional additives include thickeners (e.g., xanthan gum), emulsifiers, preservatives, flow agents (e.g., silicon dioxide), beet root powder, beta carotene, vitamins (e.g,, A, C, D, E, niacin, B6, folate, B12), minerals (e.g., iodine, magnesium, zinc, copper, manganese, chromium, fruit extracts, vegetable extracts, enzymes (e.g., amylase, cellulase, lactase, lipase, protease), legume extracts, and the like.

### III. Actions and Effects of the Energy-Promoting Compositions

Figure 1 is a flow chart that illustrates the interactive beneficial effects between the major components of the energy-promoting composition. The combination of a stimulant component, vasodilator component, and exogenous ketone body component in a single formulation provides benefits not obtainable from a composition that include only one or two of the components.

As shown, the vasodilator component beneficially functions to improve the pharmacokinetic distribution of both the ketone body component and the stimulant component. In other words, the vasodilator component may function to increase blood flow to peripheral tissues of the body so that the ketone bodies and the stimulant compound(s) can more effectively move from the digestive system to their intended targets (e.g., brain and muscle tissues).

The vasodilator component also functions to counteract the vasodilating effects of the stimulant component. Most stimulants, including caffeine, cause vasoconstriction and associated increases in blood pressure. This can be harmful to the cardiac and vascular system. Moreover, vasoconstriction reduces blood flow and thereby lowers the efficiency of pharmacokinetic delivery of the energy-promoting composition to the peripheral tissues of the body. The vasodilator component beneficially counteracts the negative vasoconstricting effects to the vascular and cardiac systems, and likewise functions to maintain effective blood flow for more efficient delivery of the energy-promoting composition to the peripheral tissues of the body.

The stimulant component also beneficially interacts with the ketone body component by enhancing pharmacokinetic absorption and uptake of the ketone bodies into the tissues so it can be readily utilized as an energy source. In other words, the stimulant component has been found to increase the efficiency in which the ketone body component is actually taken into cells and metabolized as an energy source.

The ketone body component also affects the stimulant component by attenuating the crash effect of the stimulant component. Inclusion of the ketone body component has been found to beneficially smooth out and/or prolong the experienced energy levels of the user. For example, as compared to a similar composition that omits ketone bodies or that replaces them with a sugar ingredient such as fructose, glucose, or sucrose, users report experiencing more sustained and smooth energy levels after consuming an energy-promoting composition containing the ketone body component.

Figure 1 therefore illustrates the interconnected and interactive nature of the individual components of the energy-promoting composition when combined. Each individual component enhances the beneficial effects and/or reduces the negative effects of one or both of the other components such that the combination as a whole beneficially provides a greater effect than each component individually or even two of the components combined.

Figure 2 is a chart that illustrates the expected and observed differences in energy levels experienced by a typical user when consuming a composition including caffeine ("C"), an otherwise similar composition including caffeine and a vasodilator component ("C+V"), and an otherwise similar composition including caffeine, a vasodilator component, and a ketone body component ("C+V+K") according to the invention.

As shown, the C+V composition produces a higher and faster energy peak as compared to the C composition by itself, which is due to the enhanced blood flow and enhanced delivery of caffeine to peripheral tissues. However, the C+V composition may induce a more pronounced energy crash than the C composition by itself for the same reasons it provides a more pronounced onset of effects.

In contrast, the C+V+K composition provides energy levels that substantially track the C+V composition initially. However, where the energy provided by the C+V composition begins to drop sharply, the C+V+K composition maintains a more sustained energy level due to the crash attenuating and energy sustaining properties of the ketone body component. The overall result is that the C+V+K composition provides the benefits of rapid onset and high peak in energy, coupled with higher overall energy levels over time, which minimize the crash effect relative to the C and C+V compositions.

### IV. Delivery Methods and Dosage Forms

The energy-promoting composition may be consumed or administered through any acceptable administration route. In preferred embodiments, the composition is formulated for oral consumption as a liquid "energy drink" or "energy shot." The liquid formulation may include other suitable ingredients such as flavoring agents, water, carbonated water, pH modifiers (e.g., phosphoric acid, citric acid, malic acid, tartaric acid, or succinic acid), preservatives (e.g., benzoate and/or sorbate salts), and sweeteners (e.g., sucralose, stevia saccharin, aspartame). In other embodiments the composition may be formulated as a capsule, tablet (e.g., dissolvable tablet), lozenge, powder, candy (e.g., gummy, sucker, pastille), gel, paste, mouth spray, or other dosage form suitable for oral administration. A composition having an acidic pH (e.g., between 4-5) may be desirable as a preservative and/or to provide a desirable tart or sour taste. When a beta-hydroxybutyrate mixed salt-acid and/or acetoacetate mixed salt-acid is/are used, it may be possible to manufacture a composition having a desired acidic pH that an additional pH modifier (e.g., acid) may be reduced or eliminated.

When formulated as a liquid, the energy-promoting composition may be provided in a can, bottle, flask, or other such container configured to hold a desired number of unit doses of the composition. A container may hold, for example, about 2 ounces to about 40 ounces of the energy-promoting composition, more typically about 2.5 ounces to 24 ounces or about 3 ounces to about 12 ounces. The separate components of the energy-promoting composition may be diluted or concentrated accordingly so as to provide desired levels of each component in each corresponding unit dose of the container. For example, a smaller "shot" may be more concentrated than a larger and more dilute drink.

A method of delivering an energy-promoting composition to a user may include the steps of: 1) providing an energy-promoting composition comprising a stimulant component, a vasodilator component, and an energy source including a ketone body component; and 2) administering the energy-promoting composition to the user to thereby support an energy level of the user.

An energy-promoting composition may be taken as needed to promote higher energy levels, to reduce fatigue, to enhance focus, and/or to improve mood. In some implementations it may be taken daily or in multiple doses (e.g., 2, 3, 4 or more) spread throughout the day.

### V. Examples

The following is a description of exemplary energy-promoting compositions useful for promoting higher energy levels, reducing fatigue, enhancing focus, and/or to improving mood.

### Example 1

| | |
|---|---|
| Sodium beta-hydroxybutyrate | 1 g |
| Potassium beta-hydroxybutyrate | 1 g |
| Magnesium beta-hydroxybutyrate | 1 g |
| Calcium beta-hydroxybutyrate | 1 g |
| Caffeine | 120 mg |
| Epicatechins | 500 mg |
| Huperzine-A | 200 mcg |
| Citric acid (pH modifier) | to yield pH of 5.5 |
| Flavorings | to taste |
| Water | 120 ml |

The energy-promoting composition is a drink.

### Example 2

| | |
|---|---|
| Sodium beta-hydroxybutyrate mixed salt-acid | 1 g |
| Potassium beta-hydroxybutyrate mixed salt-acid | 1 g |
| Magnesium beta-hydroxybutyrate mixed salt-acid | 1 g |
| Calcium beta-hydroxybutyrate mixed salt-acid | 1 g |
| Caffeine | 120 mg |
| Epicatechins | 500 mg |
| Huperzine-A | 200 mcg |
| Flavorings | to taste |
| pH | 5.5 |
| Water | 120 ml |

The energy-promoting composition is a drink

### Example 3

| | |
|---|---|
| Sodium beta-hydroxybutyrate mixed salt-acid | 0.35 g |
| Potassium beta-hydroxybutyrate mixed salt-acid | 2.65 g |
| Magnesium beta-hydroxybutyrate mixed salt-acid | 0-2.5 g |
| Calcium beta-hydroxybutyrate mixed salt-acid | 0-2.5 g |
| Caffeine | 150 mg |
| Methylliberine (Dynamine^{™}) | 26 mg |
| Epicatechins (Vaso 6^{™}) | 50 mg |
| Huperzine-A | 750 mcg |
| Citric acid (pH modifier) | |
| Malic Acid (pH modifier) | |
| Phosphoric Acid (pH modifier) | |
| pH | 5.5 |
| Flavorings | |
| Sucralose | |
| Potassium Sorbate | |
| Sodium Benzoate | |
| Acesulfame Potassium | |
| Water | to make 90 mL |

The energy-promoting composition is a drink

### Example 4

Any of the foregoing examples is modified to make a powder that can be added to water or other liquid to form a drink.

The claimed invention may be embodied in other specific forms without departing from its spirit or essential characteristics. The described embodiments are to be considered in all respects only as illustrative and not restrictive. The scope of the claimed invention is, therefore, indicated by the appended claims rather than by the foregoing description. All changes that come within the meaning and range of equivalency of the claims are to be embraced within their scope.

## Claims

1. A composition for promoting or supporting energy of a subject, comprising:
a stimulant component comprising one or more epinephrine and/or norepinephrine promoters selected from the group consisting of yohimbine, rauwolscine, ephedra-based compound, ephedrine, pseudoephedrine, synephrine, octopamine, 1,3-dimethylamylamine, higenamine, clenbuterol, hordenine, atomoxetine, and combinations thereof, or comprising one or more xanthine alkaloids selected from the group consisting of caffeine, theacrine, theobromine, theophylline, methylliberine, and combinations thereof;
a vasodilator component comprising one or more of quercetin, L-arginine and/or salt thereof, agmatine and/or salt thereof, gallic acid, coenzyme Q10 (CoQ10), trimethylglycine, niacin, theanine, theobromine, garlic, grape seed extract, green tea extract, catechins and/or epicatechins, proanthocyanidins, galloylated proanthocyanidins, L-norvaline, glutathione, and taurine; and
an energy source comprising an exogenous ketone body component,
wherein the exogenous ketone body component comprises a mixed salt-acid including beta-hydroxybutyric acid and at least one beta-hydroxybutyrate salt.

2. The composition of claim 1, wherein the stimulant component comprises caffeine, wherein the caffeine is provided in an amount ranging from about 30 mg to about 250 mg, or about 40 mg to about 225 mg, or about 50 mg to about 200 mg, or about 60 mg to about 180 mg, or about 80 mg to about 160 mg.

3. The composition of claim 1 or 2, wherein the stimulant component comprises one or more epinephrine and/or norepinephrine promoters, wherein the one or more epinephrine and/or norepinephrine promoters comprises yohimbine, rauwolscine, ephedra-based compound, ephedrine, pseudoephedrine, synephrine, octopamine, 1,3-dimethylamylamine, higenamine, clenbuterol, hordenine, atomoxetine, or combinations thereof.

4. The composition of any one of claims 1-3, wherein the stimulant component comprises one or more xanthine alkaloids, wherein the one or more xanthine alkaloids comprises caffeine, theacrine, theobromine, theophylline, methylliberine, or combinations thereof.

5. The composition of any one of claims 1-4, wherein the vasodilator component comprises gallic acid, niacin, theobromine, garlic, grape seed extract, green tea extract, catechins and/or epicatechins, proanthocyanidins, galloylated proanthocyanidins, L-norvaline, taurine, or combinations thereof.

6. The composition of claim 1, wherein the vasodilator component comprises one or more catechins and/or epicatechins, wherein the catechins and/or epicatechins comprises epicatechin-(4-8)-epicatechin-(4-8)-epicatechin, epicatechin-(4-8)-epicatechin-(4-8)-epicatechin-gallate, or combination thereof.

7. The composition of any one of claims 1-6, wherein the vasodilator component is included at a dose formulated to provide a vasodilating effect substantially similar to a dose of about 1 g to about 8 g citrulline, or about 2 g to about 6.5 g citrulline, or about 3 g to about 5 g citrulline.

8. The composition of any one of claims 1-7, wherein the ketone body component is included in an amount ranging from about 0.5 g to about 20 g, or about 1 g to about 15 g, or about 2 g to about 10 g, or about 3 g to about 8 g.

9. The composition of any one of claims 1-8, wherein the mixed salt-acid is formulated such that a molar ratio of sodium ions to potassium ions no greater than about about 2.5, about 2.0, or about 1.5, or even more preferably no greater than about 1.0.

10. The composition of any one of claims 1-9, wherein the composition omits glucose, fructose, and sucrose.

11. The composition of any one of claims 1-10, further comprising a nootropic component, such as wherein the nootropic component comprises one or both of a dopamine promoter and an acetylcholine promoter, preferably wherein the nootropic component comprises one or more of methylliberine, theacrine, *Mucuna pruriens,* tyrosine, L-DOPA (i.e., L-3,4-dihydroxyphenylalanine), huperzine-A, dimethylaminoethanol, phenylalanine, phenylethylamine, choline, alpha-glycerophosphocholine, uridine, citicoline, a racetam, tryptophan, 5-hydroxytryptophan (5-HTP), D-serine, phosphatidylserine, *Panax ginseng, Ginkgo biloba, Rhodiola rosea, Polygala tenuifolia, Muira puama, Eschscholzia californica, Convolvulus pluricaulis, Centella asiatica, Evolvulus alsinoides, Bacopa monnieri, Epimedium* herbs, *Ashwagandha* herbs, forskolin, vinpocetine, or a combination thereof.

12. The composition as in any one of claims 1 to 11, wherein the composition is formulated as a consumable liquid.

13. The composition of any one of claims 1 to 12, wherein the composition functions to promote one or more of wakefulness, focus, cognition, and mood.

14. The composition of any one of claims 1 to 13, wherein the energy level of the subject is greater than with an otherwise similar stimulant composition omitting one or both of the vasodilator component and the ketone body component and/or wherein the energy level of the subject is maintained for a longer duration as compared to an otherwise similar stimulant composition omitting one or both of the vasodilator component and the ketone body component.

## Patentansprüche

1. Eine Zusammensetzung zur Förderung oder Unterstützung der Energie eines Subjekts, umfassend:
eine stimulierende Komponente, die einen oder mehrere Epinephrin- und/oder Norepinephrin-Förderer umfasst, ausgewählt aus der Gruppe bestehend aus Yohimbin, Rauwolscin, einer Ephedra-basierten Verbindung, Ephedrin, Pseudoephedrin, Synephrin, Octopamin, 1,3-Dimethylamylamin, Higenamin, Clenbuterol, Hordenin, Atomoxetin und Kombinationen davon, oder die ein oder mehrere Xanthinalkaloide umfasst, ausgewählt aus der Gruppe bestehend aus Koffein, Theacrin, Theobromin, Theophyllin, Methylliberin und Kombinationen davon;
eine vasodilatierende Komponente, die einen oder mehrere der folgenden Stoffe umfasst: Quercetin, L-Arginin und/oder ein Salz davon, Agmatin und/oder ein Salz davon, Gallussäure, Coenzym Q10 (CoQ10), Trimethylglycin, Niacin, Theanin, Theobromin, Knoblauch, Traubenkernextrakt, Grünteeextrakt, Catechine und/oder Epicatechine, Proanthocyanidine, galloylierte Proanthocyanidine, L-Norvalin, Glutathion und Taurin; und
eine Energiequelle, die eine exogene Ketonkörperkomponente umfasst,
wobei die exogene Ketonkörperkomponente ein Salz-Säure-Gemisch umfasst, das Beta-Hydroxybuttersäure und mindestens ein Beta-Hydroxybutyratsalz enthält.

2. Die Zusammensetzung nach Anspruch 1, wobei die stimulierende Komponente Koffein umfasst, wobei das Koffein in einer Menge im Bereich von etwa 30 mg bis etwa 250 mg oder etwa 40 mg bis etwa 225 mg oder etwa 50 mg bis etwa 200 mg oder etwa 60 mg bis etwa 180 mg oder etwa 80 mg bis etwa 160 mg bereitgestellt wird.

3. Die Zusammensetzung nach Anspruch 1 oder 2, wobei die stimulierende Komponente einen oder mehrere Epinephrin- und/oder Norepinephrin-Förderer umfasst, wobei der eine oder die mehreren Epinephrin- und/oder Norepinephrin-Förderer Yohimbin, Rauwolscin, eine Verbindung auf Ephedra-Basis, Ephedrin, Pseudoephedrin, Synephrin, Octopamin, 1,3-Dimethylamylamin, Higenamin, Clenbuterol, Hordenin, Atomoxetin oder Kombinationen davon umfassen.

4. Die Zusammensetzung nach einem der Ansprüche 1 bis 3, wobei die stimulierende Komponente ein oder mehrere Xanthinalkaloide umfasst, wobei das eine oder die mehreren Xanthinalkaloide Koffein, Theacrin, Theobromin, Theophyllin, Methylliberin oder Kombinationen davon umfassen.

5. Die Zusammensetzung nach einem der Ansprüche 1 bis 4, wobei die vasodilatierende Komponente Gallussäure, Niacin, Theobromin, Knoblauch, Traubenkernextrakt, Grünteeextrakt, Catechine und/oder Epicatechine, Proanthocyanidine, galloylierte Proanthocyanidine, L-Norvalin, Taurin oder Kombinationen davon umfasst.

6. Die Zusammensetzung nach Anspruch 1, wobei die vasodilatierende Komponente ein oder mehrere Catechine und/oder Epicatechine umfasst, wobei die Catechine und/oder Epicatechine Epicatechin-(4-8)-Epicatechin-(4-8)-Epicatechin, Epicatechin-(4-8)-Epicatechin-(4-8)-Epicatechin-Gallat oder eine Kombination davon umfassen.

7. Die Zusammensetzung nach einem der Ansprüche 1 bis 6, wobei die vasodilatierende Komponente in einer Dosis enthalten ist, die so formuliert ist, dass sie eine vasodilatierende Wirkung bereitstellt, die im Wesentlichen derjenigen einer Dosis von etwa 1 g bis etwa 8 g Citrullin oder etwa 2 g bis etwa 6,5 g Citrullin oder etwa 3 g bis etwa 5 g Citrullin entspricht.

8. Die Zusammensetzung nach einem der Ansprüche 1 bis 7, wobei die Ketonkörperkomponente in einer Menge im Bereich von etwa 0,5 g bis etwa 20 g oder etwa 1 g bis etwa 15 g oder etwa 2 g bis etwa 10 g oder etwa 3 g bis etwa 8 g enthalten ist.

9. Die Zusammensetzung nach einem der Ansprüche 1 bis 8, wobei das Salz-Säure-Gemisch so formuliert ist, dass das molare Verhältnis von Natriumionen zu Kaliumionen nicht größer ist als etwa 2,5, etwa 2,0 oder etwa 1,5 oder, noch bevorzugter nicht größer ist als etwa 1,0.

10. Die Zusammensetzung nach einem der Ansprüche 1 bis 9, wobei die Zusammensetzung keine Glucose, Fructose und Saccharose enthält.

11. Die Zusammensetzung nach einem der Ansprüche 1 bis 10, ferner umfassend eine nootropische Komponente, wobei die nootropische Komponente beispielsweise einen Dopamin-Förderer und/oder einen Acetylcholin-Förderer umfasst, wobei die nootropische Komponente vorzugsweise eines oder mehrere von Methylliberin, Theacrin, *Mucuna pruriens*, Tyrosin, L-DOPA (d. h. L-3,4-Dihydroxyphenylalanin), Huperzin-A, Dimethylaminoethanol, Phenylalanin, Phenylethylamin, Cholin, Alpha-Glycerophosphocholin, Uridin, Citicolin, ein Racetam, Tryptophan, 5-Hydroxytryptophan (5-HTP), D-Serin, Phosphatidylserin, *Panax ginseng*, *Ginkgo biloba*, *Rhodiola rosea*, *Polygala tenuifolia, Muira puama, Eschscholzia californica, Convolvulus pluricaulis, Centella asiatica, Evolvulus alsinoides, Bacopa monnieri, Epimedium*-Kräuter, *Ashwagandha*-Kräuter, Forskolin, Vinpocetin oder eine Kombination davon, umfasst.

12. Die Zusammensetzung nach einem der Ansprüche 1 bis 11, wobei die Zusammensetzung als zum Verzehr geeignete Flüssigkeit formuliert ist.

13. Die Zusammensetzung nach einem der Ansprüche 1 bis 12, wobei die Zusammensetzung dazu dient, eines oder mehrere von Wachheit, Konzentration, Kognition und Stimmung zu fördern.

14. Die Zusammensetzung nach einem der Ansprüche 1 bis 13, wobei das Energieniveau des Subjekts höher ist als bei einer ansonsten ähnlichen stimulierenden Zusammensetzung, bei der die vasodilatierende Komponente oder die Ketonkörperkomponente oder beide fehlen, und/oder wobei das Energieniveau des Subjekts über einen längeren Zeitraum aufrechterhalten wird als bei einer ansonsten ähnlichen stimulierenden Zusammensetzung, bei der die vasodilatierende Komponente oder die Ketonkörperkomponente oder beide fehlen.

## Revendications

1. Composition pour favoriser ou soutenir l'énergie d'un sujet, comprenant:
un composant stimulant comprenant un ou plusieurs agents favorisant d'épinéphrine et/ou de norépinéphrine choisis dans le groupe constitué par la yohimbine, la rauwolscine, un composé à base d'Ephedra, l'éphédrine, la pseudoéphédrine, la synéphrine, l'octopamine, la 1,3-diméthylamylamine, l'higénamine, le clenbutérol, l'hordénine, l'atomoxétine, et des combinaisons de ceux-ci, ou comprenant un ou plusieurs alcaloïdes de xanthine choisis dans le groupe constitué par la caféine, la théacrine, la théobromine, la théophylline, la méthyllibérine, et des combinaisons de celles-ci;
un composant vasodilatateur comprenant un ou plusieurs éléments parmi la quercétine, la L-arginine et/ou un sel de celle-ci, l'agmatine et/ou un sel de celle-ci, l'acide gallique, la coenzyme Q10 (CoQ10), la triméthylglycine, la niacine, la théanine, la théobromine, l'ail, l'extrait de pépins de raisin, l'extrait de thé vert, les catéchines et/ou les épicatéchines, les proanthocyanidines, les proanthocyanidines galloylées, la L-norvaline, le glutathion et la taurine; et
une source d'énergie comprenant un composant de corps cétonique exogène,
dans laquelle le composant de corps cétonique exogène comprend un mélange sel-acide incluant de l'acide bêta-hydroxybutyrique et au moins un sel de bêta-hydroxybutyrate.

2. Composition selon la revendication 1, dans laquelle le composant stimulant comprend de la caféine, la caféine étant fournie en une quantité allant d'environ 30 mg à environ 250 mg, ou d'environ 40 mg à environ 225 mg, ou d'environ 50 mg à environ 200 mg, ou d'environ 60 mg à environ 180 mg, ou d'environ 80 mg à environ 160 mg.

3. Composition selon la revendication 1 ou 2, dans laquelle le composant stimulant comprend un ou plusieurs agents favorisant d'épinéphrine et/ou de norépinéphrine, l'un ou les plusieurs agents favorisant d'épinéphrine et/ou de norépinéphrine comprenant de la yohimbine, de la rauwolscine, d'un composé à base d'Ephedra, de l'éphédrine, de la pseudoéphédrine, de la synéphrine, de l'octopamine, de la 1,3-diméthylamylamine, de l'higénamine, du clenbutérol, de l'hordénine, de l'atomoxétine, ou des combinaisons de ceux-ci.

4. Composition selon l'une quelconque des revendications 1 à 3, dans laquelle le composant stimulant comprend un ou plusieurs alcaloïdes de xanthine, l'un ou les plusieurs alcaloïdes de xanthine comprenant de la caféine, de la théacrine, de la théobromine, de la théophylline, de la méthyllibérine, ou des combinaisons de celles-ci.

5. Composition selon l'une quelconque des revendications 1 à 4, dans laquelle le composant vasodilatateur comprend de l'acide gallique, de la niacine, de la théobromine, de l'ail, de l'extrait de pépins de raisin, de l'extrait de thé vert, des catéchines et/ou des épicatéchines, des proanthocyanidines, des proanthocyanidines galloylées, de la L-norvaline, de la taurine, ou des combinaisons de ceux-ci.

6. Composition selon la revendication 1, dans laquelle le composant vasodilatateur comprend une ou plusieurs catéchines et/ou épicatéchines, les catéchines et/ou les épicatéchines comprenant de l'épicatéchine-(4-8)-épicatéchine-(4-8)-épicatéchine, de l'épicatéchine-(4-8)-épicatéchine-(4-8)-épicatéchine-gallate, ou des combinaisons de celles-ci.

7. Composition selon l'une quelconque des revendications 1 à 6, dans laquelle le composant vasodilatateur est inclus à une dose formulée pour produire un effet vasodilatateur sensiblement similaire à celui d'une dose d'environ 1 g à environ 8 g de citrulline, ou d'environ 2 g à environ 6,5 g de citrulline, ou d'environ 3 g à environ 5 g de citrulline.

8. Composition selon l'une quelconque des revendications 1 à 7, dans laquelle le composant de corps cétonique est inclus en une quantité allant d'environ 0,5 g à environ 20 g, ou d'environ 1 g à environ 15 g, ou d'environ 2 g à environ 10 g, ou d'environ 3 g à environ 8 g.

9. Composition selon l'une quelconque des revendications 1 à 8, dans laquelle le mélange sel-acide est formulé de telle sorte que le rapport molaire des ions sodium aux ions potassium ne soit pas supérieur à environ 2,5, environ 2,0 ou environ 1,5, ou, de manière encore plus préférée, ne soit pas supérieur à environ 1,0.

10. Composition selon l'une quelconque des revendications 1 à 9, dans laquelle la composition est exempte de glucose, de fructose et de saccharose.

11. Composition selon l'une quelconque des revendications 1 à 10, comprenant en outre un composant nootropique, par exemple dans laquelle le composant nootropique comprend l'un ou les deux parmi un agent favorisant de la dopamine et un agent favorisant de l'acétylcholine, de préférence dans laquelle le composant nootropique comprend un ou plusieurs éléments parmi la méthyllibérine, la théacrine, *Mucuna pruriens*, la tyrosine, la L-DOPA (c'est-à-dire la L-3,4-dihydroxyphénylalanine), l'huperzine A, le diméthylaminoéthanol, la phénylalanine, la phényléthylamine, la choline, l'alpha-glycérophosphocholine, l'uridine, la citicoline, un racétam, le tryptophane, le 5-hydroxytryptophane (5-HTP), la D-sérine, la phosphatidylsérine, *Panax ginseng*, *Ginkgo biloba*, *Rhodiola rosea*, *Polygala tenuifolia*, *Muira puama*, *Eschscholzia californica*, *Convolvulus pluricaulis*, *Centella asiatica*, *Evolvulus alsinoides*, *Bacopa monnieri*, des herbes d'*Epimedium*, des herbes d'*Ashwagandha*, la forskoline, la vinpocétine, ou une combinaison de ceux-ci.

12. Composition selon l'une quelconque des revendications 1 à 11, dans laquelle la composition est formulée sous forme de liquide consommable.

13. Composition selon l'une quelconque des revendications 1 à 12, dans laquelle la composition agit pour favoriser l'un ou plusieurs des éléments suivants: la vigilance, la concentration, la cognition et l'humeur.

14. Composition selon l'une quelconque des revendications 1 à 13, dans laquelle le niveau d'énergie du sujet est supérieur à celui obtenu avec une composition stimulante par ailleurs similaire omettant l'un ou les deux parmi le composant vasodilatateur et le composant de corps cétonique, et/ou dans laquelle le niveau d'énergie du sujet est maintenu pendant une durée plus longue par rapport à une composition stimulante par ailleurs similaire omettant l'un ou les deux parmi le composant vasodilatateur et le composant de corps cétonique.
